# EUROPEAN PATENT APPLICATION

(11) **EP 1 842 543 A1**
(43) Date of publication of application: **10.10.2007**
(21) Application number: 06112257.8
(22) Date of filing: 05.04.2006
(51) Int. Cl.: A61K 31/437, A61K 31/585, A61K 31/565, A61P 9/04, A61P 9/12, A61P 5/42, A61P 13/12

(54) **Pharmaceutical composition coprising an aldosterone synthase inhibitor and a mineralcorticoid receptor antagonist**

(71) Applicant: Speedel Pharma AG, 4051 Basel (CH)
(72) Inventor: Schumacher, Christoph, 4123 Allschwill (CH)
(74) Representative: Maué, Paul Georg

(57) **Abstract**

The invention relates to a pharmaceutical compositions and methods of achieving a therapeutic effect including, but not limited to, the treatment of primary aldosteronism, resistant hypertension, kidney and heart disease in an animal, preferably a mammal including a human subject or a companion animal, using (i) an aldosterone synthase inhibitor or a pharmaceutically acceptable salt thereof in combination with (ii) a mineralocorticoid receptor antagonist or a pharmaceutically acceptable salt thereof and, optionally, (iii) a pharmaceutically acceptable carrier.

## Description

### FIELD OF THE INVENTION

The invention relates to a pharmaceutical compositions and methods of achieving a therapeutic effect including, but not limited to, the treatment of primary aldosteronism, resistant hypertension, kidney and heart disease in an animal, preferably a mammal including a human subject or a companion animal, using (i) an aldosterone synthase inhibitor or a pharmaceutically acceptable salt thereof in combination with (ii) a mineralocorticoid receptor antagonist or a pharmaceutically acceptable salt thereof and, optionally, (iii) a pharmaceutically acceptable carrier.

### BACKGROUND OF THE INVENTION

Aldosterone is a steroidal hormone that is mainly synthesized in the zona glomerulosa of the adrenal cortex by the enzyme aldosterone synthase (CYP11B2). The aldosterone synthase mediates the rate limiting conversion of 18-OH-corticosterone into aldosterone. The synthesis and secretion of aldosterone is controlled by angiotensin II (Ang II), potassium ion (K+) excess and sodium ion (Na+) deficiency, as well as by adrenocorticotropic hormone (ACTH) latter with only a short-lived effect. Aldosterone regulates electrolyte excretion and intravascular volume mainly through its effects on the distal tubules and cortical collecting ducts of the kidney by increasing Na+ reabsorption and K+ excretion. The state of excessive aldosterone secretion, also called hyperaldosteronism, may lead to high blood pressure, hypokalemia, alkalosis, muscle weakness, polyuria, oedamas, vasculitits, increased collagen formation, vascular remodeling, tissue fibrosis and endothelial dysfunction.

Aldosterone exerts most of its biological effects by binding to the intracellular mineralocorticoid receptor (MR) which belongs to the family of nuclear steroid receptors that also includes the glucocorticoid, progesterone, estrogen and androgen receptor. The MR is found in epithelial tissues (e.g. kidney, gastrointestinal tract, salivary and sweat glands) as well as in non-epithelial cells (heart, hippocampus, vasculature, mammary gland or leukocytes). The binding to the MR initiates the delayed or genomic responses to aldosterone, whereas faster or non-genomic responses as observed in renal, cardiac and vascular tissues appear to be mediated by other mechanisms.

Primary Aldosteronism (PA) is a clinical condition characterized by chronic, excessive and autonomous aldosterone secretion. Several causes of PA have been defined but the most frequent are aldosterone-producing adenomas (APA) and bilateral idiopathic adrenal hyperplasia (BAH). APAs are unilateral tumors of the adrenal cortex that have been originally described by Conn and also called Conn's syndrome. BAH is considered by some investigators to be a variant of essential hypertension as both adrenals secrete high amounts of aldosterone. Other causes of PA are unilateral adrenal hyperplasia, adrenal carcinomas and glucocorticoid-remediable aldosteronism.
Patients with PA display an increased susceptibility to left ventricular hypertrophy, stroke, myocardial infarction, unstable angina, arterial fibrillation, microalbuminuria, proteinuria and nephropathy. Laparoscopic adrenalectomy is regarded as the treatment of choice for APA and carcinomas. Idiopathic aldosteronism is best treated medically, since the effects of unilateral or bilateral adrenalectomy on blood pressure have usually been disappointing. In addition, after bilateral adrenalectomy patients need substitution therapy for cortisol and aldosterone deficiency.

Recent clinical evaluations indicate that primary hyperaldosteronism is common in subjects with resistant hypertension. Resistant hypertension has been conventionally defined as persistently elevated blood pressure in spite of use of three or more antihypertensive agents of different classes, one of which is a diuretic. In an evaluation of black and white patients referred with resistant hypertension a prevalence of primary hyperaldosteronism of 20% was found. These patients respond well to aldosterone blockade.

The synthesis of the spironolactone in the 1950s opened the field of pharmacologic blockade of aldosterone actions. Mineralocorticoid receptor antagonists (MRA) were shown to competitively antagonize the effects of aldosterone and deoxycorticosterone on electrolyte excretion in rats and to induce natriuresis in humans. Spironolactone of formula (I) (25 - 400 mg/day) has been the most commonly used MRA for the treatment of primary aldosteronism and other Na+-retaining conditions such as congestive heart failure (CHF), liver cirrhosis and essential hypertension.

Spironolactone also antagonizes the androgen receptor and inhibits the biosynthesis of androgens. Spironolactone is poorly absorbed after oral administration, is extensively metabolized by the liver and its therapeutic properties are attributable to the active metabolites, canrenoic acid of formula (II) and 6β-hydroxy-7α-thiomethyl spironolactone of formula (III). Endocrine side effects are therefore associated with spironolactone consisting of gynecomastia, decreased libido and impotence in men and menstrual irregularities, hirsutism and voice deepening in women. In addition, central nervous system adverse effects including drowsiness, lethargy, ataxia, confusion, headache and skin rashes have been reported. Furthermore, gastrointestinal symptoms and even breast cancer have occurred in patients taking spironolactone chronically. Therefore, it is recommended to limit the maximal dosage of spironolactone to 50 mg/day, especially in men.

Eplerenone (cf. EP 122232 A), a 9α,11 α-epoxy derivative of spironolactone of formula (IV) has recently been approved for the treatment of essential hypertension and congestive heart failure. Eplerenone is 20-fold less potent as MR antagonist compared to spironolactone, but is substantially more selective than spironolactone regarding the androgen and progesterone receptors. However, occurrence of sex hormone-related adverse events such as gynecomastia, mastodynia and vaginal bleeding has been reported and may particularly become prominent at higher drug doses needed to treat conditions of significant hyperaldosteronism. Central nervous system adverse effects include dizziness, headache and fatigue. Eplerenone metabolism is mediated via cytochrome P450 3A4 (Cyp3A4).

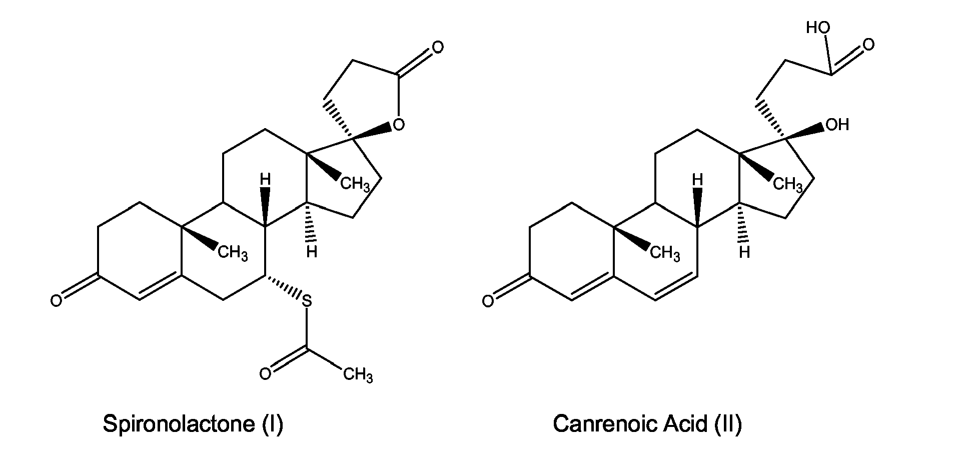

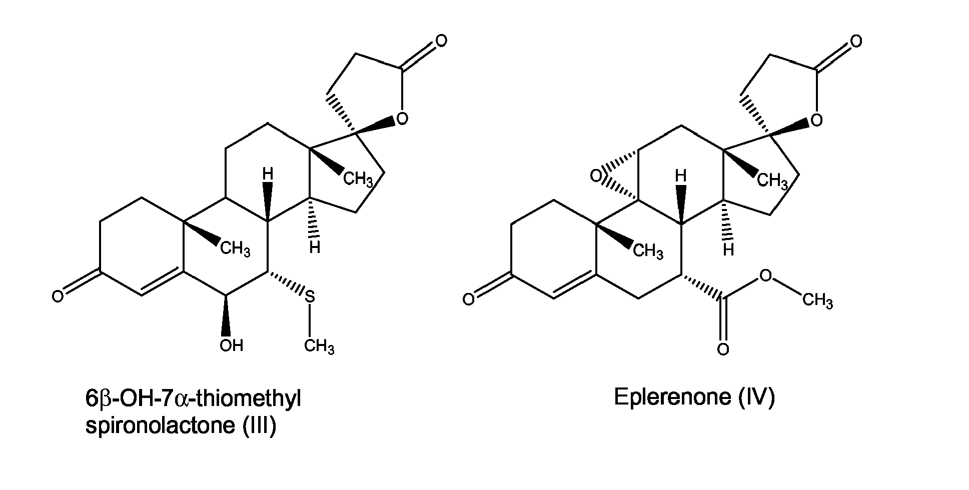

Recently, another pharmacological approach has been described to block aldosterone actions. The approach consists in inhibiting aldosterone synthase (CYP11B2), the rate-limiting and final enzymatic step for aldosterone synthesis. The family of cytochrome P450 enzymes that mediate steroid hydroxylations are distinct but structurally related proteins. The imidazoles are a class of synthetic compounds that inhibit various cytochrome P450-mediated steroid hydroxylations. Certain imidazoles derivatives have been described that exert inhibitory effects on aldosterone synthase (cf US 5057521, JP 97-071586, WO 2001/76574 A2, WO 2004/046145 A1, WO 2004/014914 A1, WO 2005/118581 A1, WO 2005/118557 A2, WO 2005/118541 A2. Fadrozole of formula (V) (cf. US patents 4617307 and 4889861), for example, is an imidazole that blocks aromatase specifically at daily dosages of 1-2 mg in postmenopausal patients with metastatic breast cancer yet at a dose of 16 mg daily produced significant suppression of both basal and ACTH-stimulated aldosterone production. This was accompanied by a significant rise in the blood 18-hydroxycorticosterone/aidosterone ratio, consistent with an aldosterone synthase inhibition.
Recently, the fadrozole enantiomer FAD286 (+)-(5R)-4-(5,6,7,8-tetrahydro-imidazo[1,5-a]pyridine-5-yl)benzonitrile hydrochloride of formula (VI) (cf. WO 2001/76574 A2) has been reported to inhibit aldosterone synthase with an IC50 of 37 nM.

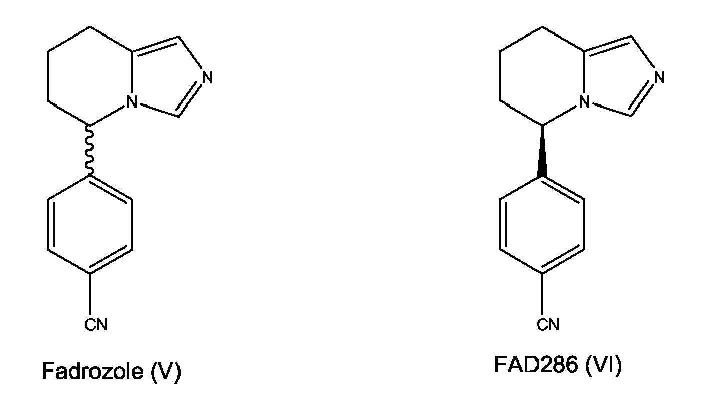

### DETAILED DESCRIPTION OF THE INVENTION

The combined administration of an aldosterone synthase inhibitor together with an mineralocorticoid receptor antagonist in conditions characterized of hyperaldosteronism such as primary aldosteronism or treatment-resistant hypertension offers the following synergistic benefits:
(i) increased efficiency to control excessive aldosterone-mediated pathologies;
(ii) Improved therapeutic profile;
(iii) Improved safety profile, for example enabling a lower dose in order to achieve a corresponding therapeutic effect, or reducing the occurrence of sex hormone-related adverse effects such as gynecomastia and impotence or the potential suppression of stress hormones and thus the stress response.

The term "synergistic" as used herein means that the effect achieved with the compositions and methods of the present invention is greater than the sum of the effects that result from compositions and methods comprising the active ingredients of this invention separately.

The person skilled in the pertinent art is fully enabled to select a relevant and standard animal test model to prove the hereinafter indicated therapeutic indications and beneficial effects.

The animal studies, for example the animal studies described hereafter, indicate that the combined administration of an aldosterone synthase inhibitor and a mineralocorticoid receptor antagonist may represent an effective therapy of conditions characterized by hyperaldosteronism such as for example primary aldosteronism and resistant hypertension.

Accordingly, the invention of combined administration of an aldosterone synthase inhibitor together with a mineralocorticoid receptor antagonist relates also to a method for the prevention of, delay of progression of, treatment of a disease or condition selected from the group consisting of primary aldosteronism of defined or undefined etiology, surgery treated primary aldosteronism, primary hyperaldosteronism, adrenal incidentalomas, essential hypertension and treatment-resistent hypertension, diabetic and non-diabetic nephropathy, congestive heart failure, renal failure, survival post-myocardial infarction, coronary heart disease, hypertension with increased formation of collagen, fibrosis, remodeling and endothelial dysfunction and diseases and conditions characterized by hyperaldosteronism or relative aldosterone excess.

The methods comprises the administration to a warm-blooded animal, including man, in need thereof a jointly effective amount of a combination of an aldosterone synthase inhibitor and a mineralocorticoid receptor antagonist.

The animal studies also indicate that the combined administration of an aldosterone synthase inhibitor together with a mineralocorticoid receptor antagonist may represent a better tolerated and therefore safer therapy of conditions characterized by hyperaldosteronism. The benefits when applying the composition of the present invention are that lower doses of the individual drugs to be combined can be used to to diminish the incidence of side effects. Lower doses may also imply that the dosages need to be applied less frequently.

The therapeutically effective amounts of the individual drugs according to the present invention can be administered simultaneously or sequentially in any order, separately or in fixed combination.

In a variation thereof, the present invention likewise relates to a "kit-of-parts", for example, in the sense that the components to be combined can be dosed independently or by use of different fixed combinations with distinguished amounts of the components. The parts of the kit of parts can then e.g. be administered simultaneously or chronologically staggered, that is at different time points and with equal or different time intervals for any part of the kit parts. Preferably, the time intervals are chosen such that the effect on the treated disease or condition in the combined use of the parts is larger and better tolerated than the effect that would be obtained by use of only one of the components.

The invention furthermore relates to a commercial package comprising the combination according to the present invention together with instructions for simultaneous, separate or sequential use.

The pharmaceutical preparations comprising the pharmacological active compound either alone or together with customary pharmaceutical auxiliary substances (carriers) are for enteral, such as oral, and rectal or parenteral administration to homeotherms.
For example, the pharmaceutical preparations may consist from 1 % to 80% of the active compound. Pharmaceutical preparations for enteral or parenteral and also for ocular administration are, for example, in unit dose forms such as coated tablets, tablets, capsules or suppositories and also ampoules. These are prepared in a manner that is known per se, for example using conventional mixing, granulation, coating, solubilizing or lyophilizing processes. Thus, the pharmaceutical preparations for oral use can be obtained and, if required or necessary, by processing the mixture or granulate into tablets or coated tablet cores after having added auxiliary substances.

The dosage of the active compound can depend on a variety of factors, such as mode of administration, homeothermic species, age and/or individual condition.

Preferred dosages for the active drugs of the pharmaceutical combination according to the present invention are therapeutically effective dosages, especially those inferior or equal to the commercial recommendation. Normally, in the case of oral administration for a patient of approximately 75 kg in weight, an approximate daily dose for an aldosterone synthase inhibitor as represented by compounds of formula (V) and (VI) may range from 0.2 mg to 200 mg per person per day, respectively from 5 to approximately 1000 mg for a mineralocorticoid receptor antagonist as represented by spironolactone of formula (I) and eplenerone of formular (IV). The daily administration may be divided preferably into 1 to 4 single doses which may, for example be of the same size. Usually children receive about half of the adult dose.

The dose necessary for each individual can be monitored, for example by measuring the serum concentration of the active drug and or the plasma steroid hormone levels and adjusted to an optimal level.

### EXAMPLES

The beneficial effects can, for example, be demonstrated in the test model as follows:

### Experimental Method

The combination according to the present invention comprising a mineralocorticoid receptor antagonist of formula (I) for example or a pharmaceutically acceptable salt thereof together with an aldosterone synthase inhibitor of formula (VI) for example or a pharmaceutically acceptable salt thereof can be administered by various routes of administration but are tested in this example using a continuous infusion via subcutaneously-implanted osmotic minipumps. Each agent can be tested over a wide range of dosages to determine the optimal drug level for each agent in combination to elicit the maximal response. Each study group is best performed in which the effects of the combination treatment group are determined at the same time as the individual components are evaluated. Although drug effects may be observed with acute administration (such as day 1), it is preferable to observe responses in a chronic setting as shown below in which experiments are done over a three to seven week observation period. The long-term duration study is of sufficient duration to allow for the full development of compensatory responses to occur and therefore, the observed effect will most likely depict the actual responses of the test system representing sustained or persistent effects. The effects on blood pressure and plasma steroid parameters as indicator of endocrine adverse effects depicted below represent a synergistic pharmacological effect when the two agents are used in combination.

### Statistical Analysis

The combination therapy can be compared to that of the monotherapy groups by determining the maximum change in plasma steroid parameters at a given blood pressure reduction in each of the treatment groups. All values are represented as the group mean ± SEM.

Statistical significance is obtained when p < 0.05. The AUC values for each of the treatment groups can be compared statistically using a one-way ANOVA followed by the appropriate post-hoc analysis, for example by performing a Tukey's test.

### Study design

The levels of aldosterone are primarily regulated by the renin-angiotensin system and potassium balance. Accordingly, mice deficient of the large conductance, voltage- and Ca²⁺-activated potassium (BK) channel present characteristics of primary aldosteronism and hypertension. This transgenic animal model is used to study the dose-dependent effects of mineralocorticod receptor antagonists and aldosterone synthase inhibitors, respectively, and in combination on plasma steroid levels as well as on blood pressure and kidney function.

BK channel-deficient (BK^{-/-}) mice are generated as described by M. Sausbier et al. Proc. Natl. Acad. Sci. 25:9474-9478, 2002. Three to four month old wild type and BK-/- mice with the hybrid SV129/C57BL6 background (always F2 generation) are used. To stimulate aldosterone production, all animals are kept on a high potassium diet (5% potassium) starting 3 weeks before administration of compounds until the end of the study. The experiments are carried out with 5 treatment groups consisting of:
- Control animals
- BK-/- plus vehicle
- BK-/- plus mineralcorticoid receptor antagonist at dose 1, 2, 3
- BK-/- plus aldosterone synthase inhibitor at dose 1, 2, 3
- BK-/- plus mineralcorticoid receptor antagonist and aldosterone synthase inhibitor at dose 1, 2

The blood pressure of the mice will be monitored continuously via chronically implanted telemetric devices. On day -10 and 21, two hours after the last administration of the compounds, blood samples will be taken from tail vein and by cardiac puncture, respectively under isoflurane anesthesia. The plasma samples are analyzed for their steroid hormone concentrations. At the end of the study, kidneys and heart of the animals will be collected. The dose 1, 2 and 3 are selected in prior pilot experiments using the Bk-/- mouse model to achieve a pharmacological effect of low, intermediate and high potency, respectively. Low is defined as a sub-therapeutic dose with minimal blood pressure reduction and no plasma steroid level change. Intermediate is defined as a therapeutic dose with optimal blood pressure reduction without plasma hormone changes. High is defined as a supra-therapeutic dose with maximal blood pressure reduction yet with plasma hormone changes. For an aldosterone synthase inhibitor of formula (V) or (VI) for example, the low dose may range from 0.05 mg/kg/day to 2 mg/kg/day, the intermediate dose from 0.2 mg/kg/day to 8 mg/kg/day and the high dose from 1 mg/kg/day to 40 mg/kg/day. Accordingly, for a mineralocorticoid receptor antagonist of formula (I) or (IV) for example, the low dose may range from 0.5 mg/kg/day to 10 mg/kg/day, the intermediate dose from 2 mg/kg/day to 40 mg/kg/day and the high dose from 10 mg/kg/day to 200 mg/kg/day.

### Plasma and urine analytes

Serum is separated from nonheparinized blood. For urine analysis, the mice are individually housed in metabolic cages and 24 hour urine collected on 1 ml HCl 6 N. The concentrations of the steroids aldosterone, corticosterone, estradiol, dihydroepiandrostendione and dihydrotestosterone were measured by radioimmunoassays. The concentrations of Na+ and K+ are measured by flame photometry.

### Results

The animal studies reveal for an aldosterone-synthase inhibitor a dose-dependent suppression of aldosterone plasma levels and aldosterone-mediated effects on blood pressure as well as cardiac and kidney function. At the supra-therapeutic doses a suppression of corticosterone plasma levels is observed. The administration of a mineralocorticoid receptor antagonist reveals a dose-dependent suppression of aldosterone-mediated effects on blood pressure as well as cardiac and kidney function in spite of an increase of plasma aldosterone levels. At the supratherapeutic doses an increase in plasma sex-steroid hormone levels is observed.

The combined administration of an aldosterone synthase inhibitor and a mineralocorticoid receptor antagonist reveal a therapeutic effect on aldosterone-mediated blood pressure as well as cardiac and kidney function at sub-therapeutic doses of the individual drugs. The individual drugs applied in combination at therapeutic doses each yield a more potent effect on aldosterone-mediated blood pressure and cardio-renal function than applied individually and the effect is not compromised by changes in plasma steroid levels i.e. stress hormones and sex-hormones. Particularly, the increase of plasma aldosterone levels upon application on a mineralocorticoid receptor antagonist are suppressed by the joint administration of the aldosterone synthase inhibitor.

## Claims

1. A pharmaceutical composition comprising
(i) an aldosterone synthase inhibitor or a pharmaceutically acceptable salt thereof;
(ii) a mineralocorticoid receptor antagonist or a pharmaceutically acceptable salt thereof; and, optionally,
(iii) a carrier.

2. Pharmaceutical combination according to claim 1 wherein said aldosterone synthase inhibitor is Fadrozole of formula (V) or FAD286 (VI) or in each case a pharmaceutically acceptable salt thereof.

3. Pharmaceutical combination according to claim 1 wherein said mineralocorticoid receptor antagonist is spironolactone of formula (I), canrenoic acid of formula (II), 6β-OH-7α-thiomethyl spironolactone of formula (III) or eplerenone of formula (IV or in each case a pharmaceutically acceptable salt thereof.

4. Pharmaceutical combination according to claim 1 wherein said mineralocorticoid receptor antagonist is spironolactone of formula (I), canrenoic acid of formula (II), 6β-OH-7α-thiomethyl spironolactone of formula (III) or eplerenone of formula (IV) and wherein said aldosterone synthase inhibitor is Fadrozole of formula (V) or FAD286 (VI) or in each case a pharmaceutically acceptable salt thereof.

5. A pharmaceutical composition according to any of claims 1 to 4 for the prevention of, delay of, progression of or treatment of a disease or condition **characterized by** hyperaldosteronism or aldosterone excess such as primary aldosteronism, primary hyperaldosteronism, adrenal incidentalomas, essential hypertension and treatment-resistent hypertension, diabetic and non-diabetic nephropathy, congestive heart failure, renal failure, survival post-myocardial infarction, coronary heart disease, hypertension with increased formation of collagen, fibrosis, remodeling and endothelial dysfunction.

6. Use of a combination comprising
(i) an aldosterone synthase inhibitor or a pharmaceutically acceptable salt thereof, and
(ii) a mineralocorticoid receptor antagonist or a pharmaceutically acceptable salt thereof for the manufacture of a medicament for the prevention of, delay of, progression of or treatment of a disease or condition **characterized by** hyperaldosteronism or aldosterone excess such as primary aldosteronism, primary hyperaldosteronism, adrenal incidentalomas, essential hypertension and treatment-resistent hypertension, diabetic and non-diabetic nephropathy, congestive heart failure, renal failure, survival post-myocardial infarction, coronary heart disease, hypertension with increased formation of collagen, fibrosis, remodeling and endothelial dysfunction.

7. A method for the prevention of, delay of, progression of or treatment of a disease or condition **characterized by** hyperaldosteronism or aldosterone excess such as primary aldosteronism, primary hyperaldosteronism, adrenal incidentalomas, essential hypertension and treatment-resistent hypertension, diabetic and non-diabetic nephropathy, congestive heart failure, renal failure, survival post-myocardial infarction, coronary heart disease, hypertension with increased formation of collagen, fibrosis, remodeling and endothelial dysfunction whereby a therapeutically effective amount of a pharmaceutical combination according to any of claims 1 to 4 is administered to a subject in need thereof.
